# EUROPEAN PATENT APPLICATION

(11) **EP 1 160 562 A1**
(43) Date of publication of application: **05.12.2001**
(21) Application number: 00870115.3
(22) Date of filing: 31.05.2000
(51) Int. Cl.: G01N 1/34, A01K 15/02

(54) **Method and apparatus for the detection of a hidden element exuding a vapor**

(71) Applicant: Universitair Bedrijvencentrum Antwerpen (UBCA), 2650 Edegem (BE)
(72) Inventor: Billet, Mic, 3010 Kessel-Lo (BE); Cox, Christophe, 3850 Nieuwerkerken (BE); Elias, Lorne, Nepean ON K2G 1R2 (CA); Van Krunkelsven, Ellen, 2640 Mortsel (BE); Verhagen, Ron, 2600 Berchem (BE); Weetjens, Bart, 2000 Antwerpen (BE)
(74) Representative: Brants, Johan Philippe Emile

(57) **Abstract**

The invention relates to a method for the on-site and in real-time detection of a hidden element which is capable of exuding at least one trace vapor component comprising the subsequential steps of:
- collecting a sample of said trace vapor component through adsorption;
- optionally, concentrating said trace vapor component sample;
- causing a forced desorption of the trace vapor component;
- delivering the desorped trace vapor content of the sample to a biosensor;
- detecting of the vapor content of the sample by a biosensor and
- finally analyzing the obtained detection result.

The invention further relates to a vapor detection device for the detection of trace vapor exuding from hidden elements comprising a caged trained animal (13) housed in a cage (22), a sniffing chamber (19) in direct contact with the cage which sniffing chamber is connected with a vapor delivery system comprising two types of samples, i.e. one type containing a known concentration of trace vapor components and a second type of samples to be detected and containing a unknown vapor concentration and a mechanism for switching the position of the two types of samples.

## Description

The present invention relates in general a method and an apparatus for the detection of a hidden element, which element is capable of exuding a trace vapor component.

A hidden element according to the present invention is a package, apparatus or device or any other item, which is as such or of which their content is at least partly not visible. In order to be detectable a hidden element according to the invention need to comprise at least one volatile component. Hidden elements can be concealed, secret, hide away or for instance buried under the ground surface. Examples of hidden elements are landmines, oil tanks, drugs contained packages, human or animal body, etc.

The present invention is primarily concerned in the detection of land mines although as stated above can be used for tracing any hidden element, which is capable of exuding trace vapor components.

In a second aspect the invention is also related to a calibration apparatus useful in the detection method and the apparatus of the invention.

The present invention provides in particular a method and apparatus for an area reduction of a suspected area for purposes of humanitarian non-destructive demining and disposal of unexploded ordnance. A major aim of the present invention is to neutralise all explosive devices in suspected areas.

Since there are only few locations within the suspected area that contain mines, time and thus operational costs can be greatly diminished when an appropriate system is used to reduce the suspected area, and to establish the borders of the actual minefields. Therefore, a highly sensitive sensor is needed that indicates the presence of trace amounts of explosive vapors, the volatile component of a landmine. Every time the sensor gives a positive signal, the borders of hazardous zones are reached. In this way the actual borders of minefields can be established. The areas that contain explosive devices can then be demined in a conventional way, using a combination of different techniques. The invention is meant to improve the existing sampling method and to integrate a quality-control of the analysing organism. A major advantage of the present invention is that it has no destructive impact on the habitat that is prospected, in contrast to the known methods which cause serious damage to the ecosystem.

### Background art

In the existing (MEDDS) system for area reduction, a vacuum pump is used (out of a mine protected vehicle) to suck the surrounding air through a filter. Explosive particles and vapors are trapped on these filters. Duplicate samples are taken, sealed, and sent to two different remote safe areas where their explosives content is analysed by trained dogs. Information is sent back to the place of operation, and areas are opened or remain closed according to the results of the sniffer dogs.

An overview article can be found on the Internet bearing as a title "Trends in landmine warfare and landmine detection" by Nardulli and Marangi, paper presented at the USPID - VII International Castiglioncello Conference on Nuclear and Conventional Disarmament: Progress or Stalemate?

Herein dogs are considered to be the best detectors of explosives. Their sensitivity to volatile explosive substances is estimated to be a factor of 10,000 higher than for a man made detector. On this consideration Mechem Consultants based their Mechem Explosives and Drugs Detection System (MEDDS). The system they developed overcomes the main problem in the use of dogs, namely the relative small period of effective work they can provide (about an hour a day). The way dogs are normally used is in the search mode, i.e. they are brought near the place where mine presence is suspected and they start searching for the source of the particular scent they have to identify. The idea underlying MEDDS method is to save dog's energies by bringing to it the source of the scent instead of using the dog in search mode. The method has been proved succesful for demining of roads . A mine resistent vehicle runs on the road with a vapour collection box endowed of chemical filters fitted on its front. Road is run along in stages of about 1 km; at each stop vapour collected from the scanned area have to be associated to that area by a physical marker or a GPS location, and a new filter is put in the collection box. Vapour collected in filters are then brought in an uncontaminated area where trained dogs start identification of explosives in the different samples. That allows to discriminate relevant sections of the roads where dogs can be used, now in search mode, for a more precise localisation of the landmine. At this stage, if the mine contains a metal part, a metal detector can be used to get the exact position. Then the mine can be digged up without exploding it to minimize damage to the road. Otherwise a fuel air charge can be used to detone it in place.

### Object of the present invention

Although the existing system reduces the search time compared to traditional methods, real-time and on-site analysis is yet not possible, since the samples are transported some distance from the area of operation for evaluation. It is therefore a main object of the present invention to provide an immediate and on-site detection method and apparatus.

A second problem in the prior art is the conflicting requirements of the sampling device. A material that adsorbs explosive vapors strongly may not release its explosive content completely, while a filter that desorbs the explosives easily is less effective to catch the explosive traces. Ideally, the sample trap should be one that adsorbs all the vapor traces while sampling, and desorbs them all while analyzing. Sample traps used in the known system are however passive filters with a high adsorption capacity, but lacking means to enhance the desorption process during analysis therefor. A second object of the present invention is therefore providing an improved desorption.

A third problem is one of quality control of the analysis. In the prior described system, two or more independent dogs are used to assess the presence of explosives in the samples. While a higher statistical probability can be obtained, no controlling measures are used to evaluate the performance of the dogs. A third object of the invention is to provide an immediate and on-site calibration device. The term immediate means in the present invention is a very short range from 5 to 15 minutes. Such a short time period is a substantial advantage of the present invention. The maximum lapse of 15 minutes from the sample taking to the result analysis makes it possible to provide a real-time detection method.

### Summary of the invention

In the present invention a method and apparatus is described for the combination of automated animal training and analysis of vapor samples, particularly from ground surfaces, by 1) collecting vapor samples by adsorption of exuding trace vapors originating from the hidden element, for example the TNT explosives from landmines; and
2) delivering the explosives content of these samples in a controlled environment. The delivering system allows calibration and an integrated quality control of the performance of the analyzing organism; and the configuration of an automated animal training setup and response feedback system for the assessment of their explosives content.

The invention is therefor related to a method for the on-site and immediate detection of a hidden element which is capable of exuding at least one trace vapor component comprising the sequential steps of:
- collecting a sample of said trace vapor component through adsorption;
- optionally, concentrating said trace vapor component sample;
- causing a forced desorption of the trace vapor component;
- delivering the desorped trace vapor content of the sample to a biosensor;
- detecting of the vapor content of the sample by a biosensor and
- finally analyzing the obtained detection result.

Preferably the detection step is followed by a calibration step, and more in particlar the calibration step comprises a forced desorption of a known trace vapor content and delivering said known content to the biosensor.

In a prefered embodiment the biosensor is a trained animal, more in particlar a rodent, preferably a rat, gerbil or a mouse.

### Detailed description of the invention

The invention will be elucidated with reference to the accompanied drawings in which:
figure 1 is a side view of a mine resistant vehicle comprising a detection apparatus according to the invention,
figure 2 is a cross sectional view of a preferred embodiment of a vapor detection device according to the invention,

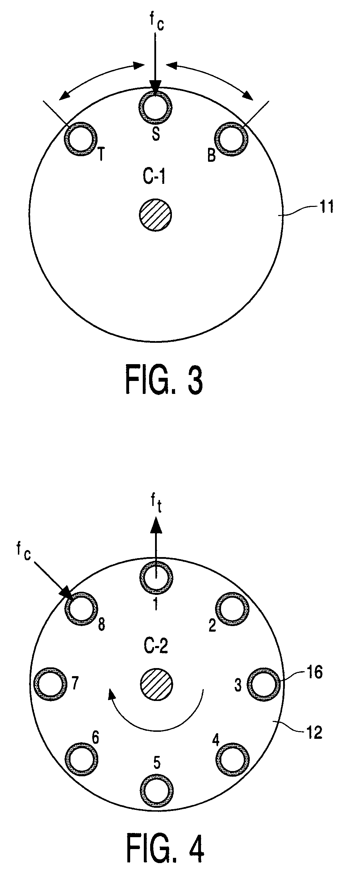

figures 3 and 4 are cross sectional views of positioned carrousel wheels C1 and C2, present in a vapor delivery system, which is an essential part of the vapor detection device, and

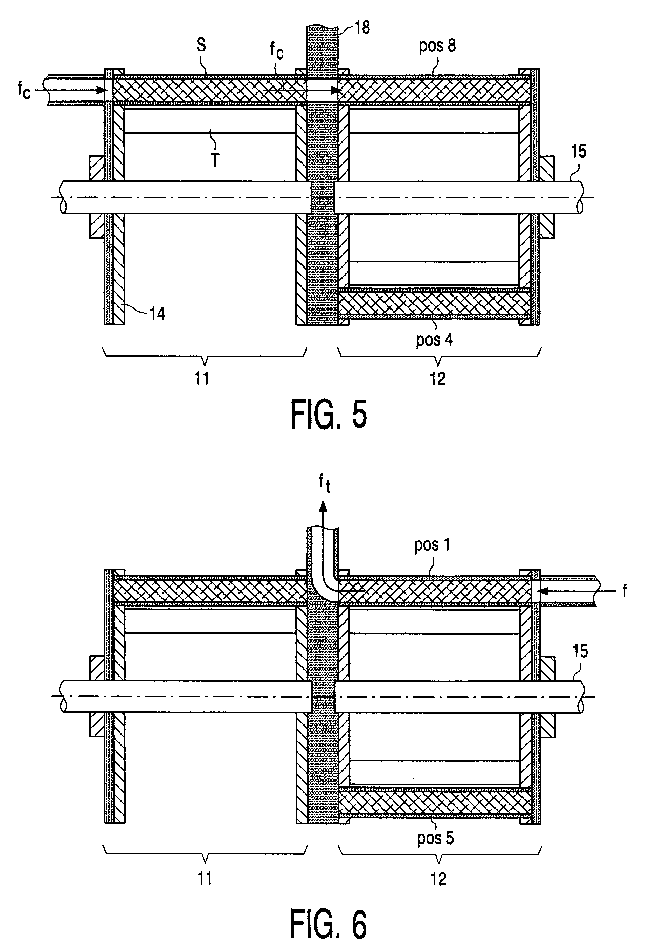

figures 5 and 6 are cross sectional views schematically explaining the forced airflow through a vapor sample in the forced desorption step of a method of the invention.

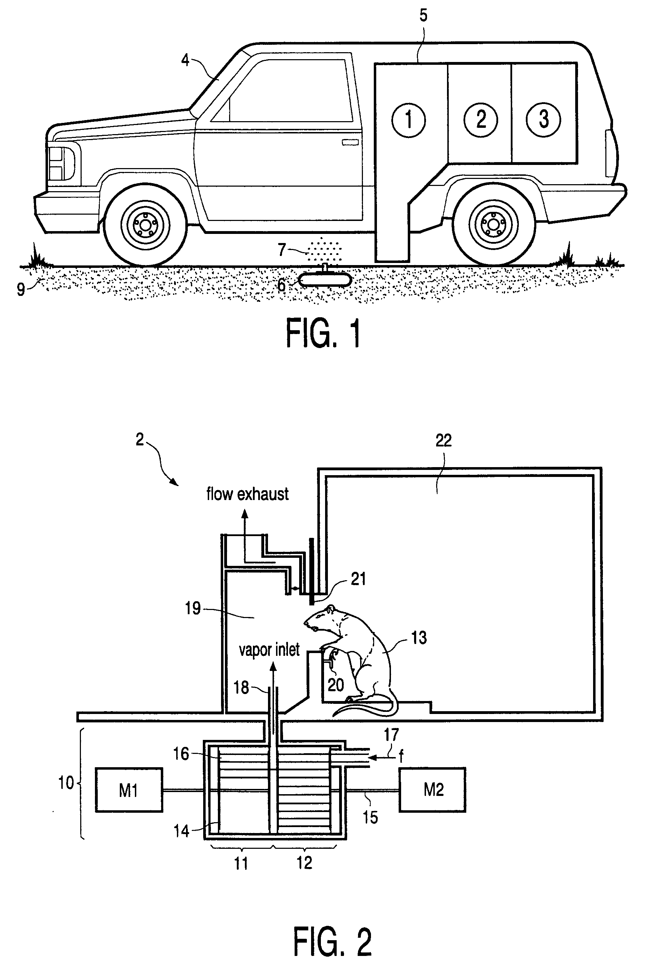

Figure 1 is a schematic view of a mine resistant vehicle 4 on a suspected area 9, which vehicle 4 comprises a combined apparatus 5 according to the invention for performing landmine detection. Said complete apparatus 5 comprises three different devices: a sampler collection device 1, a vapor detection device 2 and an analysator device 3. These three devices perform the sequential actions in the detection. The sampler collection device 1 and the analysator 3 are in general known devices. The sampler collection device 1 could comprise a vacuum pump for suction of a vapor component 7 originating from a hidden element 6 and through adsorption be caught into sample tubes. Optionally they can be further concentrated. The analysator device 3 could be a computer or a human interpreting the obtained detection results. Devices 1 and 3 are generally known in the prior art and will therefore not be disclosed in further detail.

An important feature of the vapor detection device 2 is the Vapor Delivery System, or VDS 10 (figure 2). The VDS 10 is comprised of two carousels 11-12, arranged in tandem but operating independently. Each carousel carries a number of sample tubes which provide the vapor samples for presentation to a biosensor, for example a rodent 13. Each carousel is comprised of a pair of flat circular plates 14 attached to a concentric shaft 15, the circular plates 14 being spaced about two inches apart along the shaft 15. The sample tubes 16 are held between the plates near the rims. Each carousel rotates in defined steps between matching end plates. At certain positions a particular sample tube will line up with an air line 17 which is connected to the end plates, and a small air flow (f) is used to transfer the vapor sample from the tube. The plates of the carousels are mated with their respective end plates by means of sliding, gas-tight teflon seals.

Carousel C1 (11) carries three sample tubes (16) and is used for training and calibration purposes to provide known amounts of volatile components. One tube (T) contains a small amount of TNT, one tube is a blank tube (B), and the third is the idle or standby tube (S). A metered carrier flow fc is maintained through position S. Clockwise (CW) rotation of C1 from the standby position moves the T-tube in line with the carrier stream, while counter clockwise (CCW) rotation moves the B tube in line with it. The fc exit line 18 is connected to position 8 of carousel C2.

Carousel C2 (12) carries the vapor samples in eight detachable preconcentrator (DPC) cartridges (19), and rotates CW in steps of 45 degrees. The cartridges are used for both animal training and air sampling. In the first case the DPC is either spiked -or not-through the T or B tube from the C1. In the second case the DPC is used to collect a vapor sample from the outside environment. Each cartridge incorporates a coiled length of coated metal for example nickel wire, which serves to adsorb explosive vapor and which is subsequently heated to desorb the collected vapor. Such a sample tube consisting of a tube bearing a metal wire is one of the aspects of the invention. The metal wire exhibits a better adsorption and desorption capacity for volatile components to be "trapped" on the wire. The cage 22 is separated from the sniffing chamber 19 with a vapor tight guillotine door 21.

In the training mode, a TNT vapor (or Blank) sample from C1 is loaded (spiked) onto the DPC cartridge at position 8 in C2, and held there until the appropriate signal is received. The appropriate signal is received when the rodent 13 places its nose in the chamber and interrupts a light beam, at which time C2 (12) rotates the cartridge to position 1, power is applied to the wire coil to create temperature increase and the desorbed vapors are injected into the VDS sniffing chamber 19. The desorbed vapors are carried into the chamber by means of the transfer flow ft optionally supplemented by the diluent stream F.

In the field sampling or analysis mode, C1 is not required and may be bypassed completely; or, alternatively, C1 may be operated as a calibration device to intersperse explosives, for example TNT vapor samples among the DPC field samples as refresher training for the animal, and/or as an integrated quality control for the handler. A positive signal is provoked by the rat by pushing on a lever 20, whereafter it is rewarded by an extra food supplement.

The explosive vapors on the suspected minefields will be desorbed from surface top soil samples. The surface soil samples will be heated and the vapors emanating from the sample will be sucked through the DPC, where the explosive vapors will be trapped. The device collecting the surface soil samples can be operated from within a mineproof vehicle, which allows faster collection of the soil samples. In areas which are inaccessible for vehicles the samples can be taken manually, using the necessary safety procedures and working progressively from the safe lanes.

The DPC is an intermediate carrier in the process of explosives sampling, between the environment to be sampled and the apparatus for analysis. Functionally, it is used as a means to:
1. maximally adsorb explosive particles from soil samples on a fixed, standardized carrier;
2. maximally release this explosive content at a specific time by thermal desorption.

The DPC consists of a glass tube with two end metal caps, which are connected to a coated coiled metal wire inside the glass tube. Any explosive vapors being sucked through the glass tube, will be adsorbed to the coiled metal wire as a result of the inherent high polarity of the explosive vapors.

The coiled metal wire can be heated by applying current to the connected two metal end caps. As a result, the previously adsorbed explosive vapors will be desorbed and an air stream can flush them out of the glass tube into the analysing device, in this case the rodents sniffing chamber.

A possible sequence of operation and requirements:
a. select (A) training mode or (B) analysis mode
b. In (A) training mode:
   - select ratio explosives, for example TNT/Blank random samples, range from 1/1-1/50-1/100
   - select C1 ON time (x sec.), adjustable from 2 to 10 sec in 1 sec intervals
   - start C1 operation: + signal to rotate CW to T for x sec, - signal to return to standby position (S); or - signal to rotate CCW to B for x sec, + signal to return to (S); pause for light beam signal from sniffing chamber
   - initiate one-step rotation of C2 (45 degrees) with light beam signal, turn on 24v supply to cartridge for 3 sec
   - pause for rodent response, reward, etc. Then repeat with 'start C1' operation'
c. In (B) analysis mode:
   - bypass C1 operation
   - activate cage door, pause for light beam signal from vapor chamber
   - initiate one-step rotation of C2 with light beam signal, turn on 24v supply to cartridge for 3 sec
   - pause for rodent response, reward, etc. then repeat with 'activate cage door' alternate operation:
      - manual override to allow one-shot operation for both explosives, for example TNT and blank sample of C1 as in (A) for T-sample, as an integrated quality control.

The VDS is steered by three signals:
#1: This command makes C1 turn CW
#2: This command makes C1 turn CCW
#3: This command makes C2 turn one step. C2 always turns in the same direction.

The command also activates an auxiliary power supply to heat the preconcentrator for desorption of the vapors into the sniffing chamber. Command #3 starts immediately after the rat has broken the head entry detector.

To return to the idle position after the #1 command, the #2 command is used, and vice versa, i.e., #1 and #2 are used together. The time after which C1 returns to the idle position (=time between #1 and #2) is specified as one of the time variables which are entered before the start of the training session. This variable is adjustable from 1-10 seconds in 1-second intervals.

The main advantage of the use of a double carousel is that it allows as well training as analysis in one integrated device. Hereby, the possibility is generated to control the performance of the trained animal, by randomly presenting known amounts of explosives for evaluation. Any trained animal will now and then need a maintenance retraining. Subject of the invention allows this to happen in the same device, so that for the animal's perception, there is no difference between maintenance training or operational mode. More specifically, in training mode:
1. DPC's are either neutral, or spiked with a known amount of explosives. The quantity of TNT-particles carried on the DPC is determined in function of the time Fc is blowing through the explosives container in C-1, onto the DPC in C-2. By applying the same Fc, but through the neutral container in C-1 onto the DPC in C-2, for the animal there is no other but an olfactory discrimination possible between neutral or spiked samples;
2. Once an animal has an imprint of the explosives scent, step by step the sensitivity is enhanced by gradual reducing the time of the flow Fc is applied, upto a level, where the animal perceives minimal quantities of explosives, as to be found in field samples.

In the analysis mode:
1. Field samples are being inserted for evaluation, with a randomly insertion of a spiked sample as quality control.
2. Animals that don't respond correctly to the control-samples can, without them noticing any difference, be retrained.

Rodents, in particular rats, mouse and gerbils have a highly developed olfactory capability. They have the capacity to target selected vapors out of many others. Their selectivity can exceed that of instrumental detectors. For rats, water vapor is not an interferant, as for most mechanical sensors. When trained properly, a rat responds faster then a mechanical sensor. Rats are small and lightweight, they are cheap, easy to breed and easy to maintain. They love to perform repetitive tasks, and remain strongly focussed.

Compared to the prior art, the method of sampling has been considerably improved. The invention uses detachable preconcentrator cartridges, which consist of a metal-ended glass tube, filled with a coiled length of coated nickel wire. These cartridges are used with an air pump to trap explosive vapors either (a) directly from the air above the ground area, or (b) from a heated soil sample. In the latter process much larger vapor samples can be collected on the coil. The preconcentrators, when installed in the VDS for evaluation, are thermally desorbed. In this way the explosives concentration reaching the animal's nose is enhanced by several orders of magnitude over that in the ambient air. Through the combination of maximal adsorption and thermal desorption, the conflicting requirements mentioned above is overcome.

The apparatus according to the invention is very compact. In combination with trained rats as the sensor, multiple DPC's can be evaluated by multiple rats (eventually trained on different explosives) from within a mineproof vehicle. This allows an on-site and near real-time evaluation.

The use of trained animals as a sensor for explosives detection relies very much on the performance of the animal. Animals target selected vapors among many others. Since the invention is used for training the animals as well as for evaluation of field samples, the system allows the random presentation of spiked samples (of which the exact concentration is known) to the animal. Thus, the performance of the animal can be continually monitored.

As already mentioned above, the invention is in general related to all kinds of hidden elements and not restricted to landmines.

The method and type of DPC's described above is especially designed for high molecular weight explosives, such as TNT or RDX. For the detection of other vapors different kinds of preconcentrator (metal) coils can be used. Since the selectivity of the animals is so strong, they can be trained on any vapor. As a first application there is the detection of minefields. A second application may be the analysis of trace explosive vapors exuding from postpackets, drugs, bodies and the diagnosis of some diseases as is known that some diseases provoke volatile components which can be detected with the present invention. Yet another application can be the detection of leaks in tanks.

## Claims

1. Method for the on-site and in real-time detection of a hidden element which is capable of exuding at least one trace vapor component comprising the subsequential steps of:
- collecting a sample of said trace vapor component through adsorption;
- optionally, concentrating said trace vapor component sample;
- causing a forced desorption of the trace vapor component;
- delivering the desorped trace vapor content of the sample to a biosensor;
- detecting of the vapor content of the sample by a biosensor and
- finally analyzing the obtained detection result.

2. Method according to claim 1, whereby the detection step is followed by a calibration step.

3. Method according to claim 2, whereby the calibration step comprises a forced desorption of a known trace vapor content and delivering said known content to the biosensor.

4. Method according to claims 1-3, whereby the biosensor is a trained animal.

5. Method according to claim 4, whereby the trained animal is a rodent, preferably a rat, gerbil or a mouse.

6. Method according to claims 1-5, whereby the forced desorption is performed by a forced flow of gas, for example clean or purified air which is guided over the collected sample.

7. Method according to claim 6, whereby the sample is heated for enhancing the forced desorption takes place.

8. Method according to any of the claims 1-7, wherein the hidden element is a landmine, a drug containing package, a leaking tank or a disease.

9. Device for the detection of a capable of performing a method according to the previous claims 1-8 or at least the detection step, comprising a sampler collection device, a vapor detection device and an analysator device.

10. Vapor detection device for the detection of trace vapor exuding from hidden elements comprising a caged trained animal housed in a cage, a sniffing chamber in direct contact with the cage which sniffing chamber is connected with a vapor delivery system.

11. Vapor detection device according to claim 10, whereby the cage and the sniffing chamber are separated by a vapor tight guillotine door.

12. Vapor detection device according to claim 10 or 11, comprising an air line connected to a pump for providing a forced air flow over a vapor sample.

13. Vapor detection device according to claim 10 or 12, whereby the sniffing chamber comprises a vapor inlet and a flow exhaust outlet.

14. Vapor delivery system suitable for a vapor detection device according to claim 10-13 comprising two types of samples, i.e. one type containing a known concentration of trace vapor components and a second type of samples to be detected and containing a unknown vapor concentration and a mechanism for switching the position of the two types of samples.

15. Vapor delivery system according to claim 14, whereby the vapor content of the respective samples is connected with heating means for heating the samples resulting in a forced desorption.

16. Vapor delivery system according to claims 14-15, whereby the position of the samples is controlled with a stepping motor.

17. Device for the adsorption and desorption of vapors comprising a metal wire provided in a sample tube having inlet and outlet means for allowing the flow through of a gas, for example air.

18. Method for training an animal useful in a method for the detection of hidden elements capable of exuding trace vapor components according to one of the claims 1-7, comprising:
- a first phase teaching the animal to associate a sound with a reward,
- a second phase wherein the animal is taught to make or do an action to be rewarded and
- a third phase wherein the animal will have to insert his head into the sniffing chamber before the animal can press.
